# EUROPÄISCHE PATENTSCHRIFT

(11) **EP 3 897 476 B1**
(45) Veröffentlichungstag und Bekanntmachung des Hinweises auf die Patenterteilung: **16.11.2022**
(21) Anmeldenummer: 19828613.0
(22) Anmeldetag: 12.12.2019
(51) Int. Cl.: A61F 5/01

(54) **KNIEORTHESE**
KNEE ORTHOSIS
ORTHÈSE DE GENOU

(30) Priorität: 19.12.2018 DE 102018132957
(43) Veröffentlichungstag der Anmeldung: 27.10.2021
(73) Patentinhaber: Ottobock SE & Co. KGaA, 37115 Duderstadt (DE)
(72) Erfinder: LIDOLT, Klaus, 37115 Duderstadt (DE); KRUCHEM, Tim, 37073 Göttingen (DE)
(74) Vertreter: Gramm, Lins & Partner Patent- und Rechtsanwälte PartGmbB
(86) Internationale Anmeldenummer: PCT/EP2019/084792
(87) Internationale Veröffentlichungsnummer: WO 2020/126781

(56) Entgegenhaltungen:
- DE-A1- 3 344 422
- DE-A1- 19 606 092
- GB-A- 1 290 111
- US-A- 4 681 097
- US-A1- 2006 200 057

## Beschreibung

Die Erfindung betrifft eine Knieorthese, die einen ventralen und einen dorsalen Anteil, ein Oberschenkelanlageelement, ein Unterschenkelanlageelement und ein Knieelement sowie wenigstens ein Spannelement und wenigstens einen inelastischen Gurt aufweist, wobei das Oberschenkelanlageelement und das Unterschenkelanlageelement durch ein Verbindungselement verbunden sind, durch das Druckkräfte übertragbar sind. Derartige Knieorthesen sind als Osteoarthritis-Orthesen aus dem Stand der Technik seit langem bekannt. Sie können zudem verwendet werden, um Kniefehlstellungen, insbesondere Genu varum und Genu valgum zu behandeln. Diese Fehlstellungen unterscheiden sich im Kniefrontalwinkel des Beines, also des Winkels zwischen der Längsrichtung des Oberschenkels und der Längsrichtung des Unterschenkels des Beines, der in der Frontalebene liegt. Sie werden umgangssprachlich als "X"- und "O"-Beine bezeichnet. Eine Orthese für diese Anwendungsfälle verfügt oftmals ebenfalls über ein Kniefrontalwinkel zwischen der Oberschenkelrichtung und der Unterschenkelrichtung. Auch dieser Kniefrontalwinkel liegt dann in der Frontalebene.

Entsprechende Knieorthesen sind beispielsweise aus der US 6,981,957 B2 bekannt. Sie sollen in der Lage sein, sowohl für Genu varum-Fehlstellungen als auch für Genu valgum-Fehlstellungen verwendet zu werden. Derartige Knieorthesen verfügen oftmals über Oberschenkelschienen und Unterschenkelschienen, die aus einem starren Material, beispielsweise einem Metall, hergestellt sind. Diese Schienen sind oftmals im Gelenk verbunden, dass als Schwenkgelenk ausgebildet ist, dessen Schwenkachse im angelegten Zustand der Knieorthese mit der Ausgleichsschwenkachse des Kniegelenks übereinstimmt oder zumindest nahezu übereinstimmt. Durch diese starre Ausgestaltung lässt sich zwar eine relativ große Kraft auf die unterschiedlichen Bereiche des Beines aufbringen, die Orthese ist jedoch sperrig, unbequem und hat daher nur eine geringe Akzeptanz.

Die DE 196 06 092 A1 beschreibt Gelenke, die in derartigen Knieorthesen verwendet werden können.

Aus der US 7,662,122 B2 hingegen ist eine Knieorthese bekannt, die ebenfalls über eine Dreipunktwirkung verfügt, um die benannten Fehlstellungen zu behandeln. Sie verfügt über ein Oberschenkelanlageelement und ein Unterschenkelanlageelement, die jeweils an dem Oberschenkel und dem Unterschenkel angelegt werden, um eine Druckkraft auf diesen aufzubringen. Dieses Oberschenkelanlageelement und das Unterschenkelanlageelement sind medial oder lateral, jedoch immer auf der gleichen Seite angeordnet. Auf der gegenüberliegenden Seite ist das Knieelement in Höhe des Kniegelenks des Trägers der Orthese angeordnet, um als drittes anliegendes Element eine Druckkraft auf die entgegengesetzte Richtung aufzubringen. Je nach Positionierung der einzelnen Elemente kann eine solche Orthese sowohl für die Behandlung von Genu varum-Fehlstellungen als auch für die Behandlung von Genu valgum-Fehlstellung verwendet werden.

Um die Kraft einstellen zu können, verfügt die Knieorthese über wenigstens ein Spannelement und wenigstens einen inelastischen Gurt, durch den die unterschiedlichen Elemente miteinander verbunden sind. Bei der aus der US 7,662,122 B2 bekannten Orthese ist das Spannelement in Form eines Spanndrahtes ausgebildet, dessen Länge mit einem Drehverschluss verändert werden kann. Das Element selbst verfügt über zwei Schlaufen, von denen eine das Knieelement mit dem Unterschenkelanlageelement und die andere das Knieelement mit dem Oberschenkelanlageelement verbindet. Von jeder der beiden Schlaufen verläuft ein Teil im ventralen Anteil der Knieorthese und ein anderer Teil im dorsalen Anteil. Auf diese Weise kann ein gleichmäßiger Druck von den jeweiligen Elementen auf das Bein ausgeübt werden.

Nachteilig ist jedoch, dass auf diese Weise die Positionen des Oberschenkelanlageelement und des Unterschenkelanlageelement am jeweiligen Oberschenkel oder Unterschenkel nicht einstellbar ist. Eine Verkürzung des wenigstens einen Spannelementes hat immer eine symmetrische Kraftverteilung zur Folge, so dass zwar die von der jeweiligen Pelotte auf das Bein aufgebrachte Kraft vergrößert oder verkleinert werden kann, der Kraftansatzpunkt bleibt jedoch immer gleich. Zudem ist die Knieorthese dadurch, dass die jeweiligen Spannelemente immer um das Bein des Trägers herum geführt werden müssen, konstruktiv aufwendig und damit kostenintensiv. Das Dokument US 2006/200057 beschreibt eine Knieorthese gemäß dem Oberbegriff des unabhängigen Anspruchs 1.

Der Erfindung liegt die Aufgabe zugrunde, diese Nachteile aus dem Stand der Technik zu beheben oder zumindest zu mindern. Die Erfindung löst die gestellte Aufgabe durch eine Knieorthese gemäß dem Oberbegriff des Anspruchs 1, die sich dadurch auszeichnet, dass das Oberschenkelanlageelement und das Unterschenkelanlageelement durch das wenigstens eine Spannelement im ventralen Bereich und durch den wenigstens einen inelastischen Gurt im dorsalen Bereich oder umgekehrt mit dem Knieelement verbunden sind.

Auch bei erfindungsgemäßen Knieorthesen werden folglich das Anlageelement und das Unterschenkelanlageelement durch das wenigstens eine Spannelement mit dem Knieelement verbunden. Anders als im Stand der Technik erfolgt diese Verbindung jedoch entweder im ventralen Bereich oder im dorsalen Bereich. Dies hat verschiedene Vorteile. Einerseits ist es besonders einfach möglich, die Verbindung zwischen dem Spannelement und der Element oder Anlageelement herzustellen. Schlaufenartige Spannelemente, wie sie aus dem Stand der Technik bekannt sind, sind nicht nötig. Vielmehr kann jeweils ein Ende des wenigstens einen Spannelementes an dem Oberschenkelanlageelement und ein anderes Ende an dem Unterschenkelanlageelement angeordnet werden, was beispielsweise durch einfaches Knoten, Anschrauben, Anschweißen oder sonstige Verbindung erfolgen kann. Es ist nicht notwendig, umfangreiche und lange Schlaufen gegebenenfalls im Material eines Orthesengrundkörpers anzuordnen. Hinzukommt, dass auf der jeweils gegenüberliegenden Seite, auf der das Oberschenkelanlageelement und das Unterschenkelanlageelement durch den inelastischen Gurt mit dem Knieelement verbunden sind, kein Spannelement vorhanden ist. Dies kann im ventralen Bereich von Vorteil sein, wenn der ventrale Bereich, also die Vorderseite des Beins beispielsweise im Sitzen belastet wird, haptische Eindrücke aufgenommen werden sollen oder sonstige Tätigkeiten erfolgen sollen, bei denen die Positionierung eines Spannelementes insbesondere in Form eines Spanndrahtes unbequem, gegebenenfalls sogar schmerzhaft und daher wenig akzeptiert ist. Alternativ dazu kann dies auch im dorsalen Bereich der Fall sein, was insbesondere dann von Vorteil ist, wenn die Knieorthese von einem Träger verwendet wird, der sie lange Zeit im Sitzen verwendet. Ein zu langes Sitzen auf einem beispielsweise als Spanndraht ausgebildeten Spannelement ist unbequem, kann zu schmerzhaften Hautreizungen führen und sorgt daher für eine geringe Akzeptanz der Knieorthese.

Zudem ist es durch die nur einseitige Anordnung des Spannelementes entweder auf der dorsalen Seite oder auf der ventralen Seite möglich, einseitig die Spannung zu verändern. Wird beispielsweise die Länge des Spannelementes verändert, beispielsweise verkürzt, wird nicht nur die von dem jeweiligen Element oder Anlageelement aufgebrachte Kraft verändert, sondern es kann gleichzeitig erreicht werden, dass das jeweilige Element selbst in gewissem Maße am Bein, also am Oberschenkel oder am Unterschenkel, des Trägers entlang verschoben wird, so dass sich auch der Angriffspunkt der übertragenen Kraft verändert. Dies kann, sofern dies nicht gewünscht ist, dadurch ausgeglichen werden, dass auch der wenigstens eine inelastische Gurt längenveränderbar ausgebildet ist.

Bei dem Oberschenkelanlageelement handelt es sich bevorzugt um eine Oberschenkelpelotte. Bei dem Unterschenkelanlageelement handelt es sich bevorzugt um eine Unterschenkelpelotte. Das Knieelement ist bevorzugt eine Kniepelotte.

Vorzugsweise ist das Oberschenkelanlageelement und das Unterschenkelanlageelement durch ein Verbindungselement, beispielsweise eine Stange, miteinander verbunden, durch das Druckkräfte übertragbar sind. Das Verbindungselement erstreckt sich dabei entweder auf der medialen oder auf der lateralen Seite, also auf der Seite, auf der auch das Oberschenkelanlageelement und das Unterschenkelanlageelement angeordnet sind. Auf diese Weise wird verhindert, dass die durch das Spannelement aufgebrachte Kraft dazu führt, dass das Oberschenkelanlageelement und/oder das Unterschenkelanlageelement in Richtung auf das Knie des Trägers verschoben werden.

Vorzugsweise ist das Verbindungselement in einer Richtung senkrecht zur Erstreckungsrichtung, die von dem Unterschenkelanlageelement zu dem Oberschenkelanlageelement verläuft, flexibel ausgebildet und/oder weist ein entsprechendes Gelenk auf. Auf diese Weise wird erreicht, dass das Verbindungselement, das sich über das Kniegelenk des Trägers hinauserstreckt, einer Bewegung des Knies folgen kann und diese nicht oder nur sehr gering behindert.

Vorzugsweise verfügt die Knieorthese über genau ein Spannelement, das von der Oberschenkelpelotte über die Kniepelotte zu der Unterschenkelpelotte verläuft und/oder sie verfügt über einen inelastischen Gurt, der von dem Oberschenkelanlageelement über das Knieelement zu dem Unterschenkelanlageelement verläuft. Dieses Spannelement und/oder der inelastische Gurt sind vorteilhafterweise längenveränderlich ausgebildet.

In einer bevorzugten Ausgestaltung verfügt die Knieorthese über ein Einstellelement, durch das diese Länge des wenigstens einen Spannelementes einstellbar ist, wobei sich das Einstellelement vorzugsweise an oder auf dem Knieelement befindet oder das Knieelement ist.

Bei dem Einstellelement handelt es sich beispielsweise um ein aus dem Stand der Technik an sich bekanntes Einstellelement, das beispielsweise aus der WO 2014/124054 A1 bekannt ist.

Selbstverständlich sind auch andere Einstellelemente denkbar. Insbesondere für den Fall, dass ein einziges Spannelement verwendet wird, ist die Konstruktion besonders einfach, da das eine Ende des Spannelementes an dem Oberschenkelanlageelement, das andere Ende an dem Unterschenkelanlageelement und der Einstellmechanismus an dem Knieelement angeordnet werden kann.

In einer bevorzugten Ausgestaltung verfügt die Knieorthese über wenigstens zwei Spannelemente, von denen wenigstens eines von dem Oberschenkelanlageelement zu dem Knieelement und wenigstens eines von dem Knieelement zu dem Unterschenkelanlageelement verläuft und/oder wenigstens zwei eine inelastische Gurte, von denen wenigstens einer von dem Oberschenkelanlageelement zu dem Knieelement und wenigstens eines von dem Knieelement zu dem Unterschenkelanlageelement verläuft. In einer besonders bevorzugten Ausgestaltung verfügt der inelastische Gurt über wenigstens eines, besonders bevorzugt jedoch zwei Einstellelemente zum Einstellen der Längen des inelastischen Gurtes, von denen sich besonders bevorzugt ein Einstellelement zwischen dem Oberschenkelanlageelement und dem Knieelement und das andere Einstellelement zwischen dem Knieelement und dem Unterschenkelanlageelement befindet.

Vorzugsweise verfügt jedes der Spannelemente und/oder jeder der inelastischen Gurte über ein Ende, das an einem der jeweils verbundenen Bauteile, also Knieelement, Oberschenkelanlageelement und Unterschenkelanlageelement fest montiert ist. Das jeweils andere Ende des Spannelementes und/oder des inelastischen Gurtes ist mit einem Einstellelement versehen, durch es beispielsweise aufgewickelt werden kann, um die Länge zu verkürzen. Diese Einstellelemente sind vorzugsweise an dem Knieelement angeordnet und können übereinander positioniert sein. Übereinander bedeutet dabei in diesem Fall, von dem Knie des Patienten ausgehend in radialer Richtung übereinander, also lateral oder medial nebeneinander.

Das wenigstens eine Spannelement und/oder der wenigstens eine Gurt verfügt vorzugsweise über wenigstens einen Gurt, wenigstens einen Draht, wenigstens ein Seil, wenigstens eine Zahnschienenkonstruktion und/oder einen Kabelzug. Eine Zahnschienenkonstruktion funktioniert dabei beispielsweise wie ein Ratschenmechanismus. Die Zahnschiene verfügt über eine Mehrzahl von Zähnen, die in ein Ratschenelement eingreifen. Die Schiene ist relativ zu dem Ratschenelement nur in eine Richtung bewegbar, wenn die Zähne in das Ratschenelement eingreifen, so dass beispielsweise eine Länge des mit einer Zahnschienenkonstruktion ausgebildeten Spannelementes oder inelastischen Gurtes in diesem Zustand nur verkürzbar ist. Um die Länge zu vergrößern müssen die Zähne außer Eingriff mit dem Ratschenelement gebracht werden. Dies ist beispielsweise durch Betätigen eines entsprechenden Betätigungselementes möglich.

Bevorzugt verfügt das Knieelement über wenigstens ein Schwenkgelenk, dessen Schwenkachse im angelegten Zustand der Knieorthese mit einer Schwenkachse des Knies zumindest nahezu, besonderes bevorzugt jedoch vollständig, übereinstimmt, wobei das Knieelement vorzugsweise an dem Schwenkgelenk angeordnet ist. Insbesondere für den Fall, dass das Knieelement auf dem Schwenkgelenk und das Einstellelement auf dem Knieelement angeordnet ist, ist eine besondere leichte Handhabbarkeit gewährleistet, da das Einstellelement auf diese Weise besonders leicht zugänglich ist. Die einzelnen Elemente selber können als starre, oder nahezu starre Bauteile, beispielsweise aus einem Metall, einem Kunststoff oder einem faserverstärkten Kunststoff ausgebildet sein und vorzugsweise über ein Kissenelement verfügen, das zwischen dem steifen Bauteil und der Haut des Trägers angeordnet wird.

Bevorzugt verfügt die Knieorthese über wenigstens einen, besonders bevorzugt zwei Anschläge, durch die eine Verschwenkbarkeit des Schwenkgelenkes in einer, bevorzugt in zwei Richtungen begrenzt wird. Dabei müssen die Anschläge nicht zwangsläufig symmetrisch ausgebildet sein. Es ist durchaus von Vorteil, die Flexion des Kniegelenkes in einem größeren Maß zuzulassen, als die Extension. Selbstverständlich ist auch die umgekehrte Variante möglich.

Die Knieorthese verfügt bevorzugt über einen Grundkörper, insbesondere aus einem Textil, bevorzugt aus einem Gestrick. Auf diesem können die einzelnen Elemente aufgenäht, aufgebügelt, aufgeklebt oder auf sonstige Weise aufgebracht sein. Insbesondere das wenigstens eine Spannelement verläuft bevorzugt in wenigstens einem Kanal oder Tunnel, der in dem Material des Grundkörpers angeordnet ist oder durch ein Aufbringen, insbesondere Aufnähen, Aufkleben oder Aufschweißen, einer weiteren Textillage gebildet wird. Befindet sich das wenigstens eine Spannelement innerhalb dieses Tunnels wird auf diese Weise sicher gewährleistet, dass eine versehentliche Berührung, Verstellung oder gar ein Hängenbleiben vermieden wird.

Vorzugsweise verfügt die Knieorthese über wenigstens zwei Oberschenkelanlageelemente und/oder wenigstens zwei Unterschenkelanlageelemente.

Vorzugsweise sind die beiden Anschläge an einem einzigen Anschlagsträger ausgebildet, der lösbar an einem Bauteil des Schwenkgelenkes befestigt ist. Herkömmlicherweise verfügt ein Schwenkgelenk über wenigstens zwei Bauteile, die um die Schwenkachse des Schwenkgelenkes in die beiden Schwenkrichtungen gegeneinander verschwenkbar sind. An einem der beiden Bauteile ist der Anschlagsträger angeordnet, der die beiden Anschläge trägt. Am anderen Bauteil, das gegenüber dem ersten Bauteil verschwenkbar ist, befindet sich ein Vorsprung oder Anschlagselement, das bei Erreichen eines bestimmten Verschwenkwinkels an einem der beiden Anschläge, die an dem Anschlagsträger angeordnet sind, anschlägt und auf diese Weise eine Schwenkbewegung in die jeweilige Schwenkrichtung verhindert.

Ein solches Schwenkgelenk insbesondere mit einem hier beschriebenen Anschlagsträger ist nicht nur in Knieorthesen, sondern in nahezu allen Ausgestaltungen von orthopädietechnischen Einrichtungen als Schwenkgelenk verwendbar und vorteilhaft einsetzbar. Es stellt jedoch keine eigene von einer Knieorthese losgelöste Erfindung dar. Ein Schwenkgelenk, insbesondere aber nicht notwendigerweise für eine hier beschriebene Knieorthese, wobei die Verschwenkbarkeit in einer, vorzugsweise in zwei Schwenkrichtungen durch zwei Anschläge begrenzt wird, die an einem einzigen Anschlagsträger ausgebildet sind. Ein solches Schwenkgelenk kann mit jedem einzelnen oder jeder Kombination der folgenden Merkmale ausgebildet sein, die für das Schwenkgelenk als Teil der Knieorthese beschrieben sind.

Besonders bevorzugt ist der Anschlagsträger an dem Bauteil angeklemmt, angeklipst oder angeschraubt oder in einer Ausnehmung des Bauteils eingesteckt. Unabhängig davon, welche konkrete Art der Befestigung gewählt wird, ist der Anschlagsträger lösbar ausgebildet. Dies bedeutet, dass er von dem jeweiligen Bauteil, an dem er befestigt ist, gelöst und in diesem Zustand auch entfernt werden kann. Besonders vorteilhafterweise kann er in diesem Zustand durch einen anderen Anschlagsträger mit anderen Anschlägen ausgetauscht werden. Dies ist beispielsweise dann von Vorteil, wenn bei einer bestehenden orthopädietechnischen Einrichtung, insbesondere einer bestehenden Knieorthese im Laufe der Zeit der mögliche Schwenkbereich verändert, insbesondere vergrößert werden soll. In diesem Fall ist es nicht notwendig, das Schwenkgelenk aus der orthopädietechnischen Einrichtung auszubauen und durch ein anderes Gelenk mit geändertem Schwenkbereich, also insbesondere anders positionierten Anschlägen, auszutauschen. Vielmehr ist es ausreichend, den Anschlagsträger mit den beiden Anschlägen von dem Bauteil, an dem er befestigt ist, zu lösen. In diesem Zustand kann er durch einen anderen Anschlagsträger ersetzt werden.

Die beiden Anschläge, die am Anschlagsträger angeordnet sind, können beispielsweise zwei Seitenwände eines Vorsprungs sein. Das Anschlagselement, das am anderen Bauteil des Schwenkgelenkes positioniert ist und bei Erreichen der Begrenzungsschwenkwinkel am jeweiligen Anschlag anschlägt, kommt dann bei Erreichen des einen Schwenkwinkels in der ersten Schwenkrichtung mit dem ersten Anschlag, also der ersten Seitenwand des Vorsprungs, in Kontakt. Wird das Gelenk in die entgegengesetzte Richtung verschwenkt, kommt es beim Erreichen des zweiten Anschlagswinkels zum Kontakt zwischen dem Anschlagselement und der zweiten Seitenwand des Vorsprungs am Anschlagsträger.

Durch die Ausgestaltung als lösbarer Anschlagsträger sind die verschiedenen Schwenkbereiche, die durch den jeweiligen Anschlagsträger und seine beiden Anschläge definiert werden, einfach auswählbar und dennoch reproduzierbar. Es müssen lediglich für jeden gewünschten Schwenkbereich unterschiedliche Anschlagsträger vorgesehen werden.

Mit Hilfe der beiliegenden Figuren wird nachfolgend ein Ausführungsbeispiel der vorliegenden Erfindung näher erläutert. Es zeigt:
- Figur 1: - die schematische Seitenansicht einer Knieorthese in einer medialen Draufsicht,
- Figur 2: - in einer lateralen Draufsicht und
- Figuren 3 -5: - ein Schwenkgelenk in einer Draufsicht, einer Explosionsdarstellung und einer Schwenkstellung.

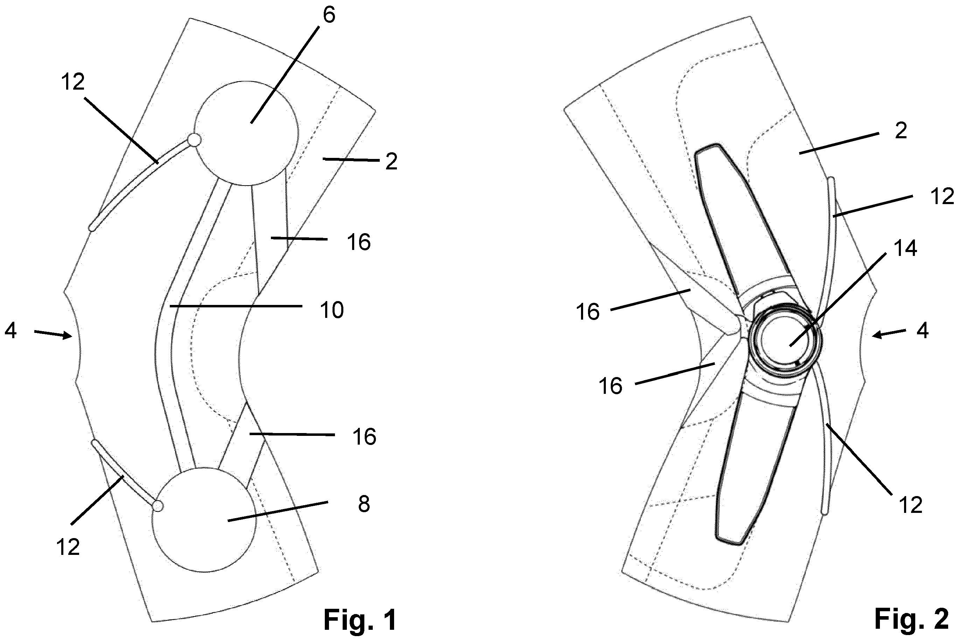

Die Figuren 1 und 2 zeigen eine Knieorthese gemäß einem Ausführungsbeispiel der vorliegenden Erfindung. Sie verfügt über einen Grundkörper 2. Der Grundkörper 2 kann aus einem elastischen, zumindest jedoch flexiblen Material, beispielsweise einem Gewebe oder Gewirk bestehen.

Der Grundkörper 2 verfügt über eine Kniescheibenöffnung 4, so dass die Kniescheibe selbst im angelegten Zustand nicht abgedeckt ist.

Figur 1 zeigt die mediale Seite der Knieorthese. Man erkennt ein Oberschenkelanlagenelement 6 und ein Unterschenkelanlagenelement 8, die jeweils am Grundkörper 2 angeordnet sind. Dazwischen erstreckt sich ein Verbindungselement 10, das Druckkräfte übertragen kann und beispielsweise eine Stabfeder ist. Sowohl am Oberschenkelanlagenelement 6 als auch am Unterschenkelanlagenelement 8 ist jeweils ein Ende eines Spannelementes 12 gezeigt. In Figur 2 ist eine Lateralansicht der Orthese dargestellt. Man erkennt ein Knieelement 14, zu dem die beiden Spannelemente 12 hingeführt sind. Selbstverständlich kann es sich auch um ein einzelnes Spannelement 12 handeln, dass das Oberschenkelanlageelement 6 über das Knieelement 14 mit dem Unterschenkelanlageelement 8 verbindet.

In Figur 1 ist zudem dargestellt, dass sowohl vom Oberschenkelanlagenelement 6 als auch vom Unterschenkelanlagenelement 8 jeweils ein inelastischer Gurt 16 wegführt. In Figur 2 ist zu erkennen, dass die inelastischen Gurte 16 zum Knieelement 14 hingeführt und dort befestigt sind. Aus den Figuren 1 und 2 wird deutlich, dass das Spannelement 12 sich ausschließlich im ventralen Bereich der Knieorthese erstreckt, während der inelastische Gurt 16 im dorsalen Bereich angeordnet ist. Selbstverständlich ist auch die umgekehrte Ausführungsform möglich.

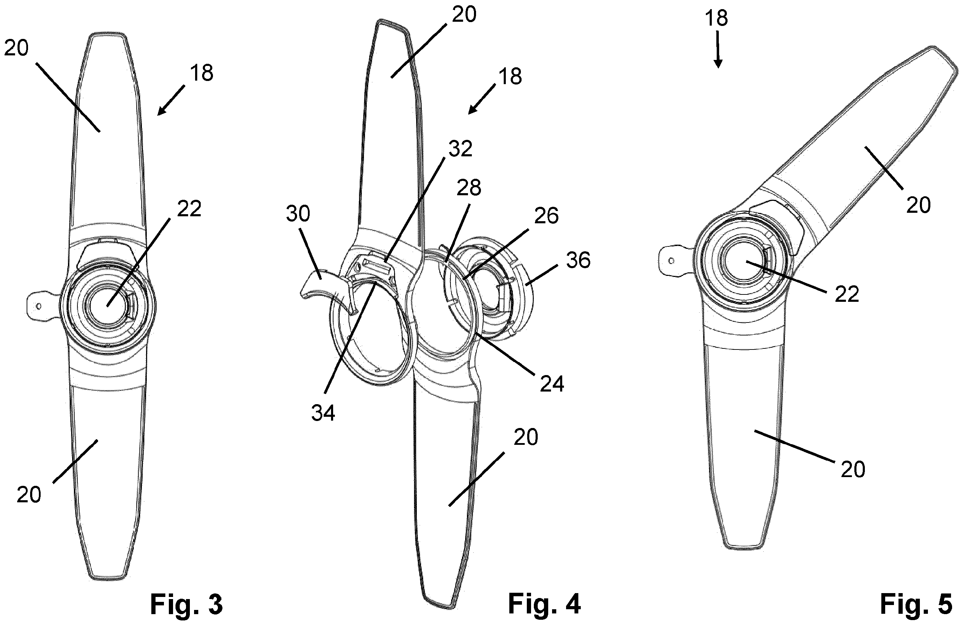

Figur 3 zeigt eine Draufsicht auf ein Schwenkgelenk 18, das insbesondere in einer hier beschriebenen Knieorthese, aber auch in anderen orthopädietechnischen Einrichtungen verwendbar ist. Es verfügt über zwei Bauteile 20, die aneinander schwenkbar angeordnet sind. In der Mitte befindet sich eine Bohrung 22, durch die sich die Schwenkachse erstreckt.

Figur 4 zeigt das Schwenkgelenk 18 aus Figur 3 in einer Explosionsdarstellung. An dem nach unten gerichteten Bauteil 20 befindet sich ein Gelenkring 24, der einen ringförmigen Schlitz 26 aufweist. Er ist an einer Stelle durch ein Anschlagselement 28 geschlossen.

Am nach oben gerichteten Bauteil 20 befindet sich ein Anschlagsträger 30, der in eine dafür vorgesehene Ausnehmung 32 am Bauteil 20 einsetzbar ist. In der Ausnehmung 32 befindet sich ein Durchbruch 34, durch den die beiden Anschläge, die in Figur 4 nicht dargestellt sind, hindurchgeführt werden können. Sie befinden sich auf der in der gezeigten Darstellung rückwärtigen Seite des Anschlagsträgers 30. Man erkennt, dass der Durchbruch 34 im montierten Zustand mit dem ringförmigen Schlitz 26 an dem anderen Bauteil 20 zur Überdeckung gelangt, sodass die Anschläge, die dich am Anschlagsträger 30 befinden und sich in die Zeichenebene hinein durch den Durchbruch 34 hindurch erstrecken, in dem Schlitz 26 angeordnet sind.

Wird nun das nach unten gerichtete Bauteil 20 relativ zum nach oben gerichteten Bauteil 20 verschwenkt, verschieben sich die durch den Durchbruch 34 hindurch ragenden Anschläge des Anschlagsträgers 30 relativ zum ringförmigen Schlitz 26 und damit auch relativ zum Anschlagselement 28. Bei bestimmten Winkelpositionen, die von dem gewählten Anschlagsträger 30 mit seinen fest montierten Anschlägen abhängen, wird das Anschlagselement 28 am jeweiligen Anschlag des Anschlagsträgers 30 zur Anlage kommen. Eine weitere Verschwenkung in die jeweilige Schwenkrichtung ist dann nicht mehr möglich, sodass der Schwenkbereich des Schwenkgelenkes 18 in dieser Form begrenzt ist.

Das in Figur 4 gezeigte Schwenkgelenk 18 verfügt zudem über ein Befestigungselement 36, an dem weitere Bauteile der orthopädietechnischen Einrichtung, beispielsweise ein Verschlusselement, angeordnet werden können.

Figur 5 zeigt das Schwenkgelenk 18 in einer relativ zu Figur 4 gezeigten Ausführung verschwenkten Position. Die beiden Bauteile 20 sind relativ zueinander um die Schwenkachse, die sich in der Bohrung 22 befindet, verschwenkt.

### Bezugszeichenliste

- 2: Grundkörper
- 4: Kniescheibenöffnung
- 6: Oberschenkelanlagenelement
- 8: Unterschenkelanlagenelement
- 10: Verbindungselement

- 12: Spannelement
- 14: Knieelement
- 16: inelastischer Gurt
- 18: Schwenkgelenk
- 20: Bauteil

- 22: Bohrung
- 24: Gelenkring
- 26: Schlitz
- 28: Anschlagselement
- 30: Anschlagsträger

- 32: Ausnehmung
- 34: Durchbruch
- 36: Befestigungselement

## Patentansprüche

1. Knieorthese, die
- einen ventralen und einen dorsalen Anteil,
- ein Oberschenkelanlageelement (6),
- ein Unterschenkelanlageelement (8) und
- ein Knieelement (14),
- wenigstens ein Spannelement (12) und wenigstens einen inelastischen Gurt (16) aufweist,
- wobei das Oberschenkelanlageelement (6) und das Unterschenkelanlageelement (8) durch ein Verbindungselement (10 verbunden sind, durch das Druckkräfte übertragbar sind
**dadurch gekennzeichnet, dass** das Oberschenkelanlageelement (6) und das Unterschenkelanlageelement (8) durch das wenigstens eine Spannelement (12) im ventralen Bereich und durch den wenigstens einen inelastischen Gurt (16) im dorsalen Bereich oder umgekehrt mit dem Knieelement (14) verbunden sind, wobei das wenigstens eine Spannelement (12) längenveränderlich ausgebildet ist und die Knieorthese ein Einstellelement aufweist, durch das eine Länge des wenigstens einen Spannelementes (12) einstellbar ist.

2. Knieorthese nach Anspruch 1, **dadurch gekennzeichnet, dass** das Verbindungselement (10) eine Stange ist.

3. Knieorthese nach Anspruch 1 oder 2, **dadurch gekennzeichnet, dass** das Verbindungselement (10) in einer Richtung senkrecht zur Erstreckungsrichtung, die von dem Unterschenkelanlageelement (8) zu dem Oberschenkelanlageelement (6) verläuft, flexibel ausgebildet ist und/oder ein Gelenk aufweist.

4. Knieorthese nach einem der vorstehenden Ansprüche, **dadurch gekennzeichnet, dass** die Knieorthese genau ein Spannelement (12) aufweist, das von dem Oberschenkelanlageelement (6) über das Knieelement (14) zu dem Unterschenkelanlageelement (8) verläuft und/oder genau einen inelastischen Gurt (16) aufweist, der von dem Oberschenkelanlageelement (6) über das Knieelement (14) zu dem Unterschenkelanlageelement (8) verläuft.

5. Knieorthese nach einem der vorstehenden Ansprüche, **dadurch gekennzeichnet, dass** das Einstellelement an oder auf dem Knieelement (14) befindet oder das Knieelement (14) ist.

6. Knieorthese nach einem der vorstehenden Ansprüche, **dadurch gekennzeichnet, dass** die Knieorthese wenigstens zwei Spannelemente (12) aufweist, von denen wenigstens eines von dem Oberschenkelanlageelement (6) zu dem Knieelement (14) und wenigstens eines von dem Knieelement (14) zu dem Unterschenkelanlageelement (8) verläuft und/oder wenigstens zwei inelastische Gurte (16) aufweist, von denen wenigstens einer von dem Oberschenkelanlageelement (6) zu dem Knieelement (14) und wenigstens einer von dem Knieelement (14) zu dem Unterschenkelanlageelement (8) verläuft.

7. Knieorthese nach einem der vorstehenden Ansprüche, **dadurch gekennzeichnet, dass** das wenigstens eine Spannelement (12) und/oder der wenigstens eine inelastische Gurt (16) wenigstens einen Gurt, wenigstens einen Draht, wenigstens ein Seil, wenigstens eine Zahnschienenkonstruktion, und/oder einen Kabelzug aufweist.

8. Knieorthese nach einem der vorstehenden Ansprüche, **dadurch gekennzeichnet, dass** das Knieelement (14) wenigstens ein Schwenkgelenk (18) aufweist, dessen Schwenkachse im angelegten Zustand der Knieorthese mit einer Schwenkachse des Knies zumindest nahezu übereinstimmt, wobei das Knieelement (14) vorzugsweise an dem Schwenkgelenk (18) angeordnet ist.

9. Knieorthese nach Anspruch 8, **dadurch gekennzeichnet, dass** die Knieorthese wenigstens einen, bevorzugt zwei Anschläge aufweist, durch die eine Verschwenkbarkeit des Schwenkgelenkes (18) in einer, bevorzugt in zwei Schwenkrichtungen, vorzugsweise in der Sagittalebene, begrenzt wird.

10. Knieorthese nach einem der vorstehenden Ansprüche, **dadurch gekennzeichnet, dass** sie wenigstens zwei Oberschenkelanlageelemente (6) und/oder wenigstens zwei Unterschenkelanlageelemente (8) aufweist.

11. Knieorthese nach Anspruch 9 oder 10, **dadurch gekennzeichnet, dass** das die beiden Anschläge an einem einzigen Anschlagsträger (30) ausgebildet sind, der lösbar an einem Bauteil des Schwenkgelenkes (18) befestigt ist.

12. Knieorthese nach Anspruch 11, **dadurch gekennzeichnet, dass** der Anschlagsträger an dem Bauteil angeklemmt, angeklipst, oder angeschraubt oder in eine Ausnehmung des Bauteils eingesteckt ist.

## Claims

1. A knee orthosis which comprises
- a ventral and a dorsal section,
- an upper leg contact element (6),
- a lower leg contact element (8) and
- a knee element (14),
- at least one bracing element (12) and at least one inelastic strap (16),
- wherein the upper leg contact element (6) and the lower leg contact element (8) are connected by a connecting element (10), by way of which compressive forces can be transmitted,
**characterized in that** the upper leg contact element (6) and the lower leg contact element (8) are connected to the knee element (14) by the at least one bracing element (12) in the ventral region and by the at least on inelastic strap (16) in the dorsal region, or vice-versa, wherein the at least one bracing element (12) is designed to be adjustable in length and the knee orthosis features an adjustment element by way of which a length of the at least one bracing element (12) can be adjusted.

2. The knee orthosis according to claim 1, **characterized in that** the connecting element (10) is a rod.

3. The knee orthosis according to claim 1 or 2, **characterized in that** the connecting element (10) is designed to be flexible in a direction perpendicular to the direction of extension running from the lower leg contact element (8) to the upper leg contact element (6) and/or has a corresponding joint.

4. The knee orthosis according to one of the preceding claims, **characterized in that** the knee orthosis comprises precisely one bracing element (12) that extends from the upper leg contact element (6) via the knee element (14) to the lower leg contact element (8) and/or precisely one inelastic strap (16) that extends from the upper leg contact element (6) via the knee element (14) to the lower leg contact element (8).

5. The knee orthosis according to one of the preceding claims, **characterized in that** the adjustment element is situated on or at the knee element (14) or is the knee element (14).

6. The knee orthosis according to one of the preceding claims, **characterized in that** the knee orthosis has at least two bracing elements (12), of which at least one extends from the upper leg contact element (6) to the knee element (14) and at least one extends from the knee element (14) to the lower leg contact element (8) and/or at least two inelastic straps(16), of which at least one extends from the upper leg contact element (6) to the knee element (14) and at least one extends from the knee element (14) to the lower leg contact element (8).

7. The knee orthosis according to one of the preceding claims, **characterized in that** the at least one bracing element (12) and/or the at least one inelastic strap (16) comprises at least one strap, at least one wire, at least one cable, at least one toothed rail construction and/or a cable pull.

8. The knee orthosis according to one of the preceding claims, **characterized in that** the knee element (14) has at least one swivel joint (18) whose swivel axis in the mounted state of the knee orthosis at least almost coincides with a swivel axis of the knee joint, wherein the knee element (14) is preferably arranged on the swivel joint (18).

9. The knee orthosis according to claim 8, **characterized in that** the knee orthosis features at least one, but preferably two end stops, by way of which a swivelling of the swivel joint (18) is limited in one, but preferably two directions, preferably in the sagittal plane.

10. The knee orthosis according to one of the preceding claims, **characterized in that** it comprises at least two upper leg contact elements (6) and/or at least two lower leg contact elements (8).

11. The knee orthosis according to claim 9 or 10, **characterized in that** the two end stops are configured on a single end stop carrier (30), which is fastened to a component of the swivel joint (18) such that it can be detached.

12. The knee orthosis according to claim 11, **characterized in that** the end stop carrier is clamped, clipped or screwed onto the component, or plugged in a recess of the component.

## Revendications

1. Orthèse de genou comportant
- une partie ventrale et une partie dorsale,
- un élément d'appui de cuisse (6),
- un élément d'appui de bas de jambe (8) et
- un élément de genou (14),
- au moins un élément tendeur (12) et au moins une sangle inélastique (16),
- l'élément d'appui de cuisse (6) et l'élément d'appui de bas de jambe (8) étant reliés par un élément de liaison (10) permettant de transmettre des forces de pression,
**caractérisée en ce que**
l'élément d'appui de cuisse (6) et l'élément d'appui de bas de jambe (8) sont reliés à l'élément de genou (14) par ledit au moins un élément tendeur (12) dans la zone ventrale et par ladite au moins une sangle inélastique (16) dans la zone dorsale, ou inversement, ledit au moins un élément tendeur (12) étant réalisé de façon variable en longueur, et l'orthèse de genou comprenant un élément de réglage qui permet de régler une longueur dudit au moins un élément tendeur (12).

2. Orthèse de genou selon la revendication 1,
**caractérisée en ce que**
l'élément de liaison (10) est une tige.

3. Orthèse de genou selon la revendication 1 ou 2,
**caractérisée en ce que**
dans une direction perpendiculaire à la direction d'extension qui s'étend de l'élément d'appui de bas de jambe (8) à l'élément d'appui de cuisse (6), l'élément de liaison (10) est réalisé de façon flexible et/ou présente une articulation.

4. Orthèse de genou selon l'une des revendications précédentes, **caractérisée en ce que**
l'orthèse de genou présente exactement un élément tendeur (12) qui s'étend de l'élément d'appui de cuisse (6) à l'élément d'appui de bas de jambe (8) en passant par l'élément de genou (14), et/ou présente exactement une sangle inélastique (16) qui s'étend de l'élément d'appui de cuisse (6) à l'élément d'appui de bas de jambe (8) en passant par l'élément de genou (14).

5. Orthèse de genou selon l'une des revendications précédentes, **caractérisée en ce que**
l'élément de réglage se situe sur ou au niveau de l'élément de genou (14) ou est l'élément de genou (14).

6. Orthèse de genou selon l'une des revendications précédentes, **caractérisée en ce que**
l'orthèse de genou présente au moins deux éléments tendeurs (12), dont l'un au moins s'étend de l'élément d'appui de cuisse (6) à l'élément de genou (14) et dont l'autre au moins s'étend de l'élément de genou (14) à l'élément d'appui de bas de jambe (8), et/ou présente au moins deux sangles inélastiques (16), dont l'une au moins s'étend de l'élément d'appui de cuisse (6) à l'élément de genou (14) et dont l'autre au moins s'étend de l'élément de genou (14) à l'élément d'appui de bas de jambe (8).

7. Orthèse de genou selon l'une des revendications précédentes, **caractérisée en ce que**
ledit au moins un élément tendeur (12) et/ou ladite au moins une sangle inélastique (16) comprend au moins une sangle, au moins un fil, au moins une corde, au moins une construction de crémaillère, et/ou un câble de commande.

8. Orthèse de genou selon l'une des revendications précédentes, **caractérisée en ce que**
l'élément de genou (14) comprend au moins une articulation pivotante (18) dont l'axe de pivotement, à l'état appliqué de l'orthèse de genou, coïncide au moins presque avec un axe de pivotement du genou, l'élément de genou (14) étant de préférence disposé sur l'articulation pivotante (18).

9. Orthèse de genou selon la revendication 8,
**caractérisée en ce que**
l'orthèse de genou comprend au moins une, de préférence deux butées, par lesquelles une capacité de pivotement de l'articulation pivotante (18) est limitée dans une, de préférence dans deux directions de pivotement, de préférence dans le plan sagittal.

10. Orthèse de genou selon l'une des revendications précédentes, **caractérisée en ce que**
elle présente au moins deux éléments d'appui de cuisse (6) et/ou au moins deux éléments d'appui de bas de jambe (8).

11. Orthèse de genou selon la revendication 9 ou 10,
**caractérisée en ce que**
les deux butées sont formées sur un seul support de butée (30) qui est fixé de manière amovible à un composant de l'articulation pivotante (18).

12. Orthèse de genou selon la revendication 11,
**caractérisée en ce que**
le support de butée est fixé audit composant par serrage, par clipsage ou par vissage, ou est inséré dans un évidement dudit composant.
